# EUROPEAN PATENT SPECIFICATION

(11) **EP 3 132 267 B1**
(45) Date of publication and mention of the grant of the patent: **14.08.2019**
(21) Application number: 15780576.3
(22) Date of filing: 17.04.2015
(51) Int. Cl.: G01N 33/574, G01N 33/566

(54) **METHOD TO SCREEN OUT FALSE POSITIVES IN A CIRCULATING TUMOR CELL ASSAY**
VERFAHREN ZUM AUSSIEBEN VON FALSCH-POSITIVEN IN EINEM TEST ZIRKULIERENDER TUMORZELLEN
PROCÉDÉ POUR CRIBLER DES FAUX POSITIFS DANS UN TEST DE CELLULES TUMORALES EN CIRCULATION

(30) Priority: 17.04.2014 US 201461980760 P
(43) Date of publication of application: 22.02.2017
(73) Proprietor: Siemens Healthcare Diagnostics Inc., Tarrytown, NY 10591 (US)
(72) Inventor: PUGIA, Michael, Granger Indiana 46540 (US); PHILIP, Julia, South Bend Indiana 46615 (US); MARFURT, Karen, Edwardsburg Michigan 49112 (US)
(74) Representative: Schweitzer, Klaus
(86) International application number: PCT/US2015/026438
(87) International publication number: WO 2015/161231

(56) References cited:
- WO-A1-2014/037552
- US-A1- 2011 306 043
- US-A1- 2011 306 043
- US-A1- 2013 129 681

## Description

### TECHNICAL FIELD

Provided herein are methods of screening a patient sample in a circulating tumor cell assay for false positives and methods of performing circulating tumor cell detection assays. Also provided herein are uses of those methods in the treatment of a patient.

### BACKGROUND

Cellular analysis is important in medical applications, such as diagnosis of many diseases. Many medical applications of cellular analysis, however, require isolation of certain cells of interest which typically represent only a small fraction of the analyzed sample. For example, circulating tumor cells (hereinafter referred to as "CTCs") are of particular interest in the diagnosis of metastatic cancers. In conventional methods, CTCs are isolated from whole blood by first removing red blood cells by lyses. In a 10 ml blood sample, a few hundred CTCs are to be separated from about 800,000,000 white blood cells (hereinafter "WBC"). This requires methods with high separation efficiency and cell recovery rates. For CTCs to be analyzed by conventional scanning microscopy methods or molecular methods such as next generation sequencing, for example, normal cells (typically WBC) must be reduced to 200 to 1 diseased cell (typically cancer cells) and the sample volume must be reduced from 10 ml to 100s of microliters. Detection of the CTCs may be further complicated due to the presence of normal cells (i.e. non-CTCs) in the sample, which can interfere with the accuracy of the analysis.

US2013/0129681 A1 is directed to a process for determining the signaling capability of bladder cancer cells where whole blood is used and hematopoietic cells are excluded from epithelial cells using CD45. Then, tumor type specific markers are used in order to separate tumor cells, such as CTCs from other cells and epithelial markers are used in order to separate normal cells from tumor cells.

WO2014/037552 A1 is directed to a method for analyzing the composition of circulating tumor cell (CTC) population in a biological sample comprising the steps a) isolation or enrichment of peripheral blood mononuclear cells (PMBCs) and CTCs from the biological sample, b) depleting hematopoietic cells from the isolated or enriched PMBCs and CTCs, c) testing the obtained cell suspension for at least two markers independently from each other selected from epithelia markers, mesenchymal markers, epithel-to- mesenchymal transition (EMT) markers, surface markers, tissue markers, tumor markers, markers for resistance, stem cell markers, hematopoietic markers and d) thereby identifying one or more subgroup(s) of CTCs in the CTC population of that biological sample.

US2011/306043 A1 discloses that useful endothelial cell surface markers include CD105, CD106, CD144, and CD146.

Thus, there is a need for methods of screening out false positives in circulating tumor cell assays.

### SUMMARY

The invention in its broadest form is defined by independent claims 1, 3 and 4. Preferred embodiments are defined in dependent claims 2 and 5-10.

Provided herein are methods of screening a patient sample in a circulating tumor cell assay for false positives.

Also provided herein are methods of performing a circulating tumor cell detection assay.

Also provided herein are methods of simultaneously identifying, and methods of distinguishing between, a circulating tumor cell and a circulating endothelial cell in a patient sample.

### BRIEF DESCRIPTION OF THE DRAWINGS

The summary, as well as the following detailed description, is further understood when read in conjunction with the appended drawings. For the purpose of illustration, there are shown in the drawings exemplary embodiments of the disclosed methods. In addition, the drawings are not necessarily drawn to scale. In the drawings:

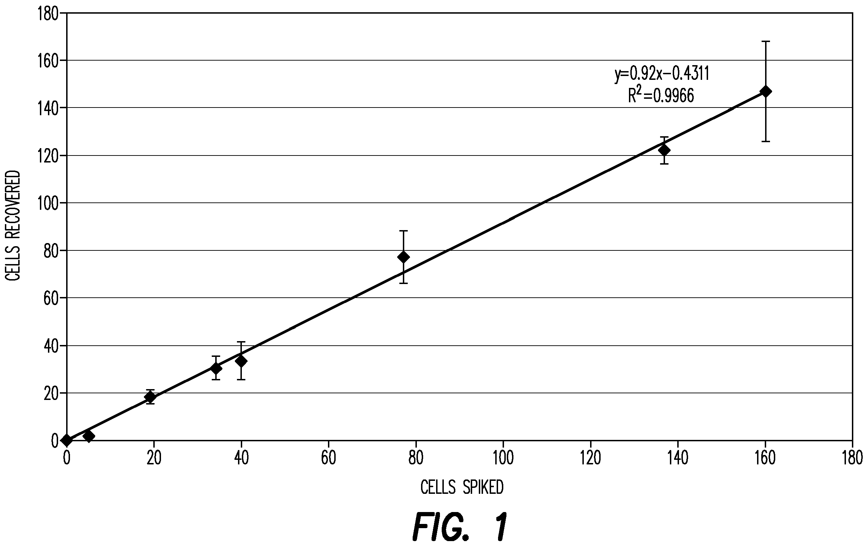

FIG 1 represents a line graph showing CTC recovery using fixed cancer cells from tissue culture spiked into blood from healthy donors.

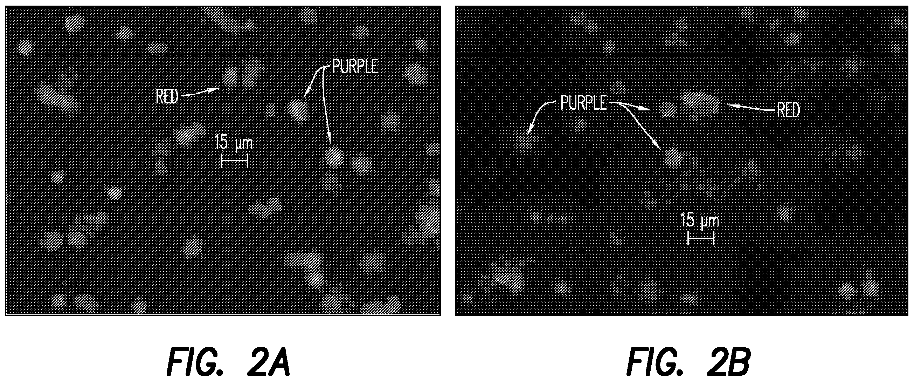

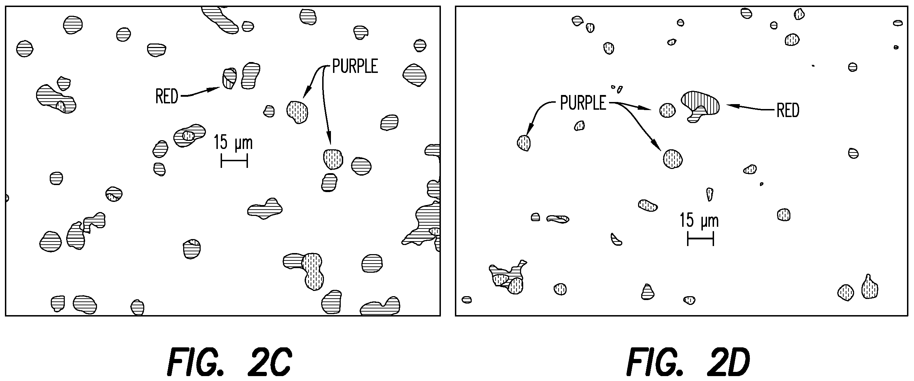

FIG 2, comprising FIGS 2A, 2B, 2C and 2D represents an overlay of immunofluorescence images from CTC recovery in blood samples from cancer patients. The samples were analyzed with antibodies to vimentin (red) and CK8/18/19 (red) as cancer cell markers and antibodies to CD45 (purple) as a white blood cell marker. DAPI (blue) was used to stain cellular nulei.

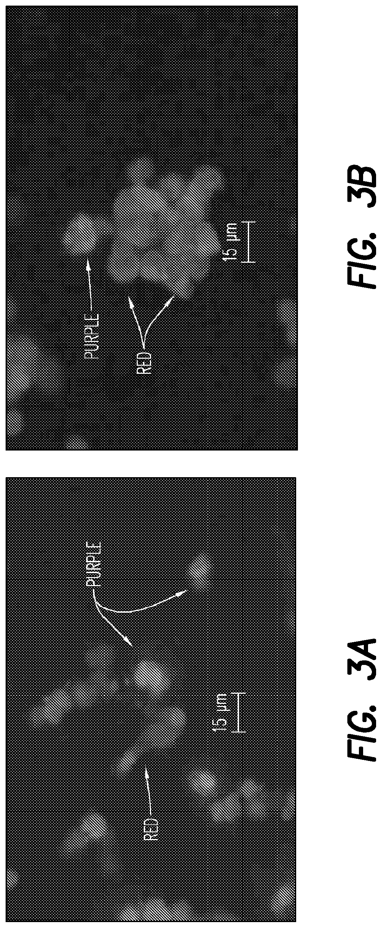

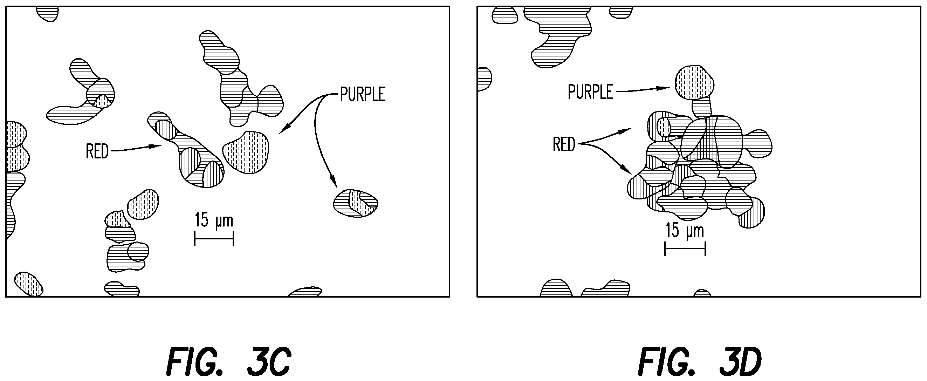

FIG 3, comprising FIGS 3A, 3B, 3C and 3D represents an overlay of immunofluorescence images showing false positives found in blood samples from healthy donors. The samples were analyzed with antibodies to vimentin (red) and CK8/18/19 (red) as cancer cell markers and antibodies to CD45 (purple) as a white blood cell marker. DAPI (blue) was used to stain cellular nulei.

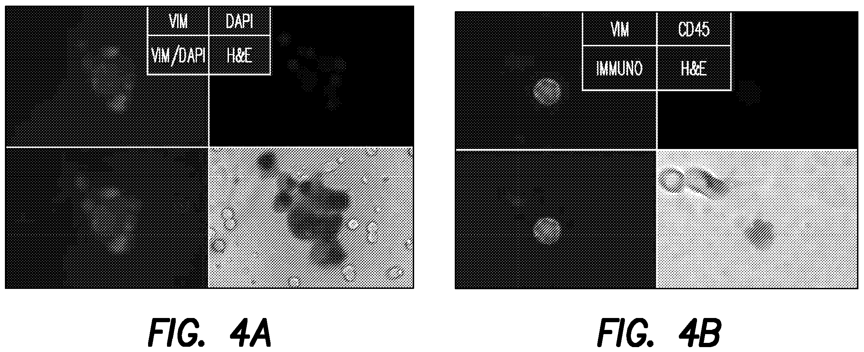

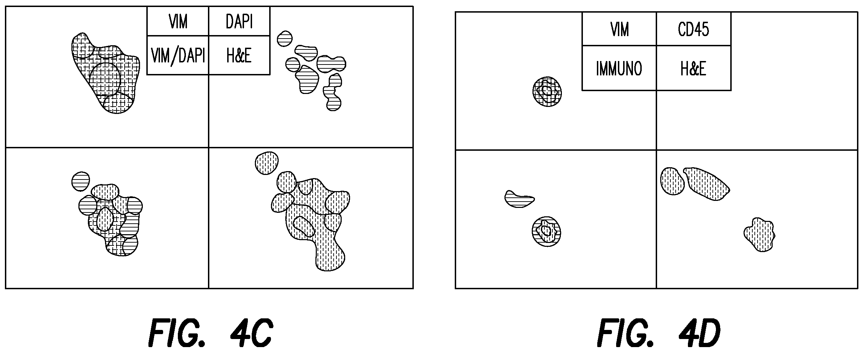

FIG 4, comprising FIGS 4A, 4B, 4C and 4D represents immunocytochemistry (ICC) using anti-vimentin antibodies and hematoxylin and eosin (H&E) staining of (A) and (C) filtered cancer cells and (B) and (D) false positives. (A) and (C) Vimentin (top left), DAPI (top right), overlay of vimentin and DAPI (bottom left) and H&E staining (bottom right). (B) and (D) Vimentin (top left), CD45 (top right), overlay of vimentin and DAPI (bottom left) and H&E staining (bottom right).

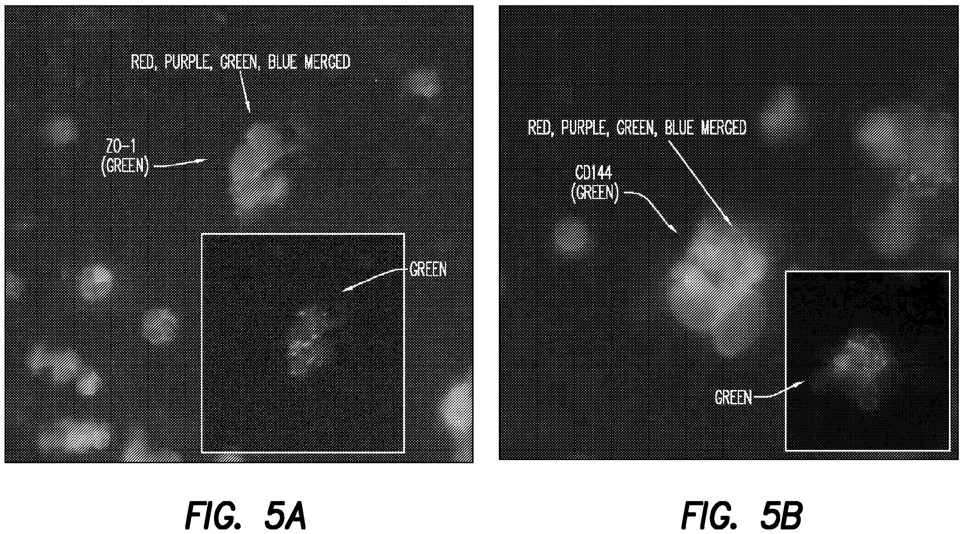

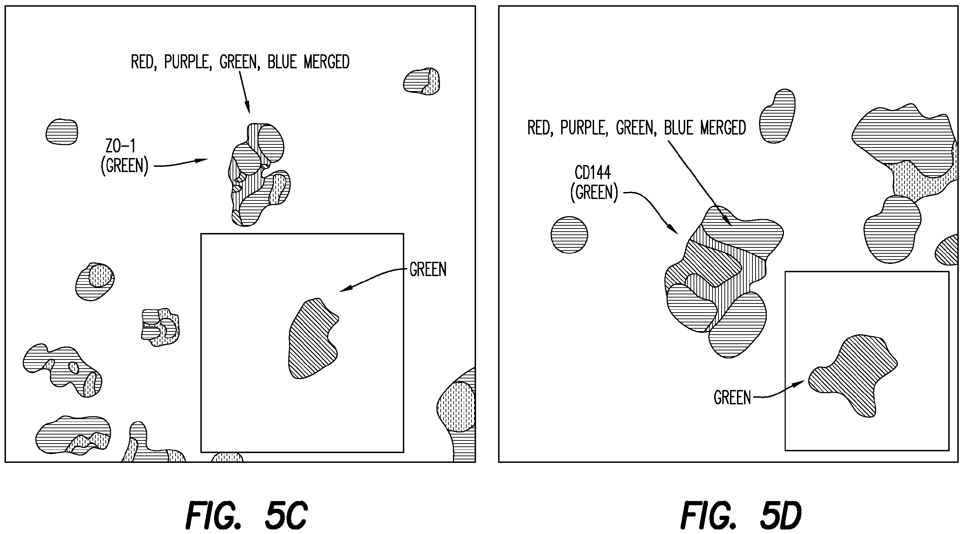

FIG 5, comprising FIGS 5A, 5B, 5C and 5D represents overlays of immunofluorescence images from blood from healthy donors stained with anti-vimentin (red), anti-CD45 (purple) and (A) and (C) anti-ZO-1 (green) or (B) and (D) anti-CD 144 (green) monoclonal antibodies. DAPI (blue) was used to stain cellular nulei.

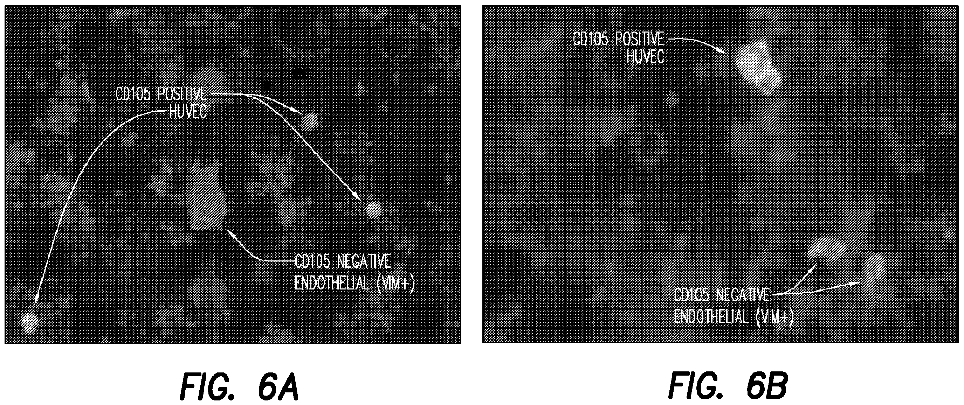

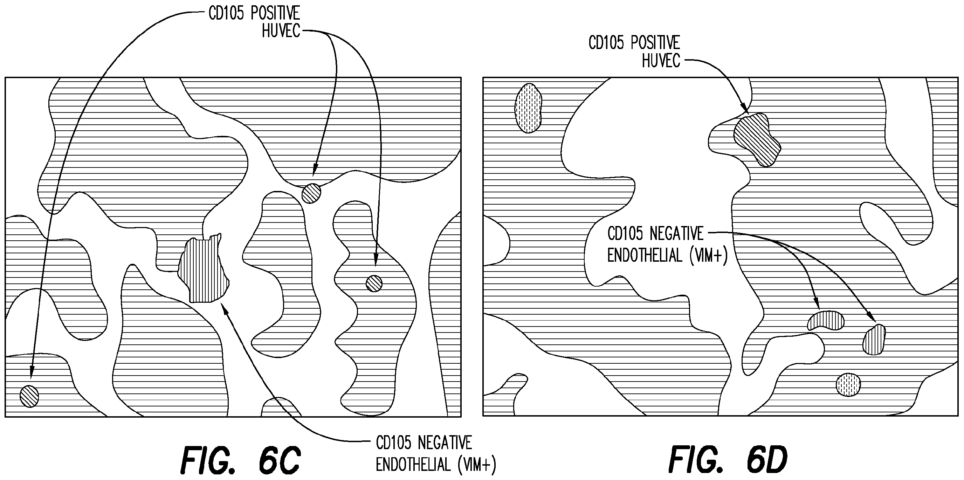

FIG 6, comprising FIGS 6A, 6B, 6C and 6D represents overlays of immunofluorescence images from HUVEC cells spiked into blood from healthy donors stained with anti-vimentin (red), anti-CK8/18/19 (red), anti-CD45 (purple) and anti-CD 105 (green) monoclonal antibodies. DAPI (blue) was used to stain cellular nulei.

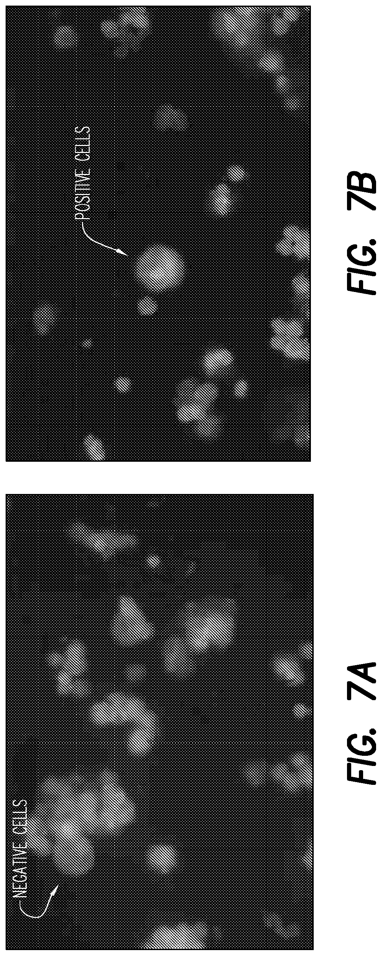

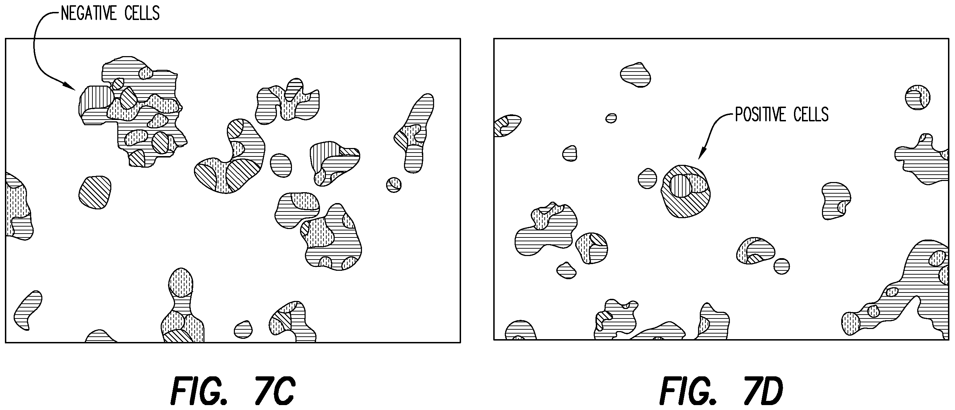

FIG 7, comprising FIGS 7A, 7B, 7C and 7D represents overlays of a tyramide signal amplification (TSA) assay. Filtered and fixed cells (HUVEC cells spiked into blood from healthy donors) were incubated with primary antibodies to vimentin (red), CK8/18/19 (red), and CD45 (purple), incubated with an anti-rabbit HRP secondary antibody, and incubated with tyramine-Alexa488 to perform the signal amplification. TSA response is green. DAPI (blue) was used to stain cellular nulei. Negative cells did not stain green. Positive cells stained green.

### DETAILED DESCRIPTION OF THE ILLUSTRATIVE EMBODIMENTS

The disclosed methods may be understood more readily by reference to the following detailed description taken in connection with the accompanying figures and examples, which form a part of this disclosure. It is to be understood that the disclosed methods are not limited to the specific methods, conditions or parameters described and/or shown herein, and that the terminology used herein is for the purpose of describing particular embodiments by way of example only and is not intended to be limiting of the claimed methods. Also, as used in the specification including the appended claims, the singular forms "a," "an," and "the" include the plural, and reference to a particular numerical value includes at least that particular value, unless the context clearly dictates otherwise. The term "plurality", as used herein, means more than one. When a range of values is expressed, another embodiment includes from the one particular value and/or to the other particular value. Further, reference to values stated in ranges include each and every value within that range. Similarly, when values are expressed as approximations, by use of the antecedent "about," it will be understood that the particular value forms another embodiment. All ranges are inclusive and combinable.

It is to be appreciated that certain features of the disclosed methods which are, for clarity, described herein in the context of separate embodiments may also be provided in combination in a single embodiment. Conversely, various features of the disclosed methods that are, for brevity, described in the context of a single embodiment may also be provided separately or in any subcombination.

Provided herein are methods as claimed in the appended claims.

As used herein, the term "patient" refers to any mammal whose sample can be analyzed with the disclosed methods. Thus, the disclosed methods are applicable to human and nonhuman subjects, although it is most preferably used for humans. In some embodiments, the patient sample is a human sample. In other embodiments, the patient sample is a nonhuman sample. "Patient" and "subject" are used interchangeably herein.

As used herein, the term "sample" refers to a blood sample, which can contain, for example, normal cells and CTCs. In some embodiments, the blood sample can be whole blood. In other embodiments, the blood sample can be plasma.

Samples can be collected from the patient into a suitable container such as, but not limited to, a bag, a bottle, a needle or a VACUTAINER® container.

As used herein, "circulating tumor cell" (CTC) refers to cells that have shed, dislodged, and/or metastasized from primary tumor and entered into the bloodstream.

As used herein, the term "false positive" refers to non-CTCs which appear as CTCs in a screening assay. False positives include, but are not limited to, red blood cells (RBCs), platelets, and non-cancerous WBCs, endothelial cells, epithelial cells, mesenchymal cells, stem cells, or any combination thereof. "Non-CTCs," are also referred to as "normal cells" or "non-cancerous cells" herein.

CTCs are distinguished from false positives by incubating the sample with a plurality of antibodies, wherein the plurality of antibodies comprises an antibody that binds to vimentin, an antibody that binds to another marker expressed on circulating tumor cells, an antibody that binds to a marker expressed on white blood cells, and an antibody that binds to a marker expressed on endothelial cells.

Markers expressed on CTCs include, but are not limited to, vimentin, basic cytokeratins, neutral cytokeratins, acid cytokeratins, adhesion molecules, or any combination thereof. Basic or neutral cytokeratins include, but are not limited to, CK1, CK2, CK3, CK4, CK5, CK6, CK7, CK8 and CK9. Acidic cytokeratins include, but are not limited to, CK10, CK12, CK 13, CK14, CK16, CK17, CK18, CK19 and CK20. In the present invention, the another marker expressed on CTCs comprises CK8, CK18, CK19, or any combination thereof. These types of cancer cell type biomarkers may be utilized alone or in combination with any of the other cancer cell type biomarkers including, but are not limited to, WAF, BAX-1, PDGF, JAGGED 1, NOTCH, VEGF, VEGFR, CAIX, MIB1, MDM, PR, ER, SEL5, SEMI, PI3K, AKT2, TWIST1, EML-4, DRAFF, C-MET, ABL1, EGFR, GNAS, MLH1, RET, MEK1, AKT1, ERBB2, HER2, HNF1A, MPL, SMAD4, ALK, ERBB4, HRAS, NOTCH 1, SMARCB1, APC, FBXW7, IDH1, NPM1, SMO, ATM, FGFR1, JAK2, NRAS, SRC, BRAF, FGFR2, JAK3, RA, STK11, CDH1, FGFR3, KDR, PIK3CA, TP53, CDKN2A, FLT3, KIT, PTEN, VHL, CSF1R, GNA11, KRAS, PTPN11, DDR2, CTNNB1, GNAQ, MET, RBI, AKT1, BRAF, DDR2, MEK1, NRAS, FGFR1, and ROS1. These markers may be specific to the CTCs (i.e. not expressed on normal cells) or may be expressed on both CTCs and normal cells. As used herein, "another marker expressed on circulating tumor cells" refers to CTC markers other than vimentin. Thus, in some embodiments, the antibody that binds to another marker expressed on CTCs is an anti-CK8 antibody. In other embodiments, the antibody that binds to another marker expressed on CTCs is an anti-CK18 antibody. In other embodiments, the antibody that binds to another marker expressed on CTCs is an anti-CK19 antibody. In yet other embodiments, the antibody that binds to another marker expressed on CTCs is a combination of anti-CK8, anti-CK18, and/or anti-CK19 antibodies, including, but not limited to, anti-CK8/18/19 antibodies.

Markers expressed on WBCs include, but are not limited to CD45, CTLA-4, CD4, CD68 and/or CD8. Thus, in some embodiments, the marker expressed on white blood cells comprises anti-CD45 antibodies. Additionally, CD45 can be used to differentiate the different types of white blood cells when combined with other CD markers. For example, granulocytes can be indicated by CD45+, CD15+; monocytes can be indicated by CD45+, CD14+; T lymphocytes can be indicated by CD45+, CD3+; T helper cells can be indicated by CD45+,CD3+, CD4+; cytotoxic T cells can be indicated by CD45+,CD3+, CD8+; B lymphocytes can be indicated by CD45+, CD19+ or CD45+, CD20+; thrombocytes can be indicated by CD45+, CD61+; and natural killer cells can be indicated by CD16+, CD56+, CD3-. Furthermore, two commonly used CD molecules, CD4 and CD8, can be used as markers for helper and cytotoxic T cells, respectively. These molecules are defined in combination with CD3+, as some other leukocytes also express these CD molecules (i.e., some macrophages express low levels of CD4; dendritic cells express high levels of CD8).

The marker expressed on endothelial cells is CD144.

Antibodies can be prepared by techniques that are well known in the art such as immunization of a host and collection of sera (polyclonal) or by preparing continuous hybrid cell lines and collecting the secreted protein (monoclonal) or by cloning and expressing nucleotide sequences or mutagenized versions thereof coding at least for the amino acid sequences required for specific binding of natural antibodies.

Antibodies include a complete immunoglobulin or fragment thereof, including the various classes and isotypes, such as IgA, IgD, IgE, IgG1, IgG2a, IgG2b and IgG3, and IgM. Fragments thereof include Fab, Fv and F(ab')2, and Fab'. In addition, aggregates, polymers, and conjugates of immunoglobulins or their fragments can be used where appropriate so long as binding affinity for a particular molecule is maintained.

Also provided herein are methods of simultaneously identifying a circulating tumor cell and a circulating endothelial cell in a patient sample, comprising:
incubating the sample with a plurality of antibodies, wherein the plurality of antibodies comprises an antibody that binds to vimentin, an antibody that binds to CK8/18/19, and an antibody that binds to CD144;
detecting the plurality of antibodies, wherein the detecting comprises generating signals from the plurality of antibodies; and
   identifying the presence of a circulating tumor cell and a circulating endothelial cell in the sample comprising:
   screening for a circulating tumor cell, wherein the circulating tumor cell has a signal from the antibody that binds to vimentin, a signal from the antibody that binds to CK8/18/19, or both, but not a signal from the antibody that binds to CD144; and
   screening for a circulating endothelial cell, wherein the circulating endothelial cell has a signal from the antibody that binds to vimentin, a signal from the antibody that binds to CK8/18/19, or both, and a signal from the antibody that binds to CD144.

Also provided herein are methods for distinguishing between a circulating tumor cell and a circulating endothelial cell in a patient sample, comprising:
incubating the sample with a plurality of antibodies, wherein the plurality of antibodies comprises an antibody that binds to vimentin, an antibody that binds to CK8/18/19, and an antibody that binds to CD144;
detecting the plurality of antibodies, wherein the detecting comprises generating signals from the plurality of antibodies; and
distinguishing between a circulating tumor cell and a circulating endothelial cell, comprising screening the sample for a circulating tumor cell, wherein the circulating tumor cell has a signal from the antibody that binds to vimentin, a signal from the antibody that binds to CK8/18/19, or both, but not a signal from the antibody that binds to CD144, and screening the sample for a circulating endothelial cell, wherein the circulating endothelial cell has a signal from the antibody that binds to vimentin, a signal from the antibody that binds to CK8/18/19, or both, and a signal from the antibody that binds to CD144.

In some embodiments, the CTC has a signal from the antibody that binds to vimentin but not a signal from the antibody that binds to CD144. In other embodiments, the CTC has a signal from the antibody that binds to CK8/18/19 but not a signal from the antibody that binds to CD144. In yet other embodiments, the CTC has a signal from the antibody that binds to vimentin and a signal from the antibody that binds to CK8/18/19 but not a signal from the antibody that binds to CD144.

In some embodiments, the circulating endothelial cell (CEC) has a signal from the antibody that binds to vimentin and a signal from the antibody that binds to CD144. In other embodiments, the CEC has a signal from the antibody that binds to CK8/18/19 and a signal from the antibody that binds to CD144. In other embodiments, the CEC has a signal from the antibody that binds to vimentin, a signal from the antibody that binds to CK8/18/19, and a signal from the antibody that binds to CD 144.

As used herein, the term "detecting" refers to identifying the antibody that is bound to vimentin, the antibody that is bound to another marker expressed on CTCs, the antibody that is bound to a marker expressed on white blood cells, and/or the antibody that is bound to a marker expressed on endothelial cells, by, for example, generating signals from the bound antibodies.

Signals can be generated from the bound antibodies by a number of techniques known in the art. In some embodiments the plurality of antibodies contain one or more detectable labels. The nature of the label is dependent on the particular assay format. A signal producing system usually includes one or more components, at least one component being a detectable label, which generates a detectable signal that relates to the amount of bound and/or unbound label, i.e., the amount of label bound or not bound to the cell being detected or to an agent that reflects the amount of the cell to be detected. The one or more detectable labels can be any molecule that produces, or can be induced to produce, a signal, including, but not limited to fluorescent tags, radiolabels, enzyme labels, chemiluminescent labels, photosensitizer labels, or any combination thereof. Thus, detecting the plurality of antibodies can comprise detecting and/or measuring, radioactivity, enzyme activity, luminescence, light absorbance, or any combination thereof.

Suitable labels include, for example: dyes; fluorescers such as fluorescein, isothiocyanate, rhodamine compounds, phycoerythrin, phycocyanin, allophycocyanin, o phthaldehyde, and fluorescamine; enzymes such as alkaline phosphatase, glucose-6-phosphate dehydrogenase ("G6PDH"), β-galactosidase, and horseradish peroxidase; ribozyme; a substrate for a replicase such as QB replicase; promoters; complexes such as those prepared from CdSe and ZnS present in semiconductor nanocrystals known as Quantum dots; chemiluminescers such as isoluminol and acridinium esters, for example; sensitizers; coenzymes; enzyme substrates; radiolabels such as ¹²⁵l, ¹³¹l, ¹⁴C, ³H, ⁵⁷ Co and ⁷⁵Se; particles such as latex particles, carbon particles, metal particles including magnetic particles, e.g., chromium dioxide (CrO₂) particles, and the like; metal sol; crystallite; liposomes; cells, etc., which may be further labeled with a dye, catalyst or other detectable group.

The label can directly produce a signal and, therefore, additional components are not required to produce a signal. Numerous organic molecules, for example fluorescers, are able to absorb ultraviolet and visible light, where the light absorption transfers energy to these molecules and elevates them to an excited energy state. This absorbed energy is then dissipated by emission of light at a second wavelength. Other labels that directly produce a signal include radioactive isotopes and dyes.

Alternatively, the label may need other components to produce a signal, and the signal producing system would then include all the components required to produce a measurable signal. Such other components may include substrates, coenzymes, enhancers, additional enzymes, substances that react with enzymatic products, catalysts, activators, cofactors, inhibitors, scavengers, metal ions, and a specific binding substance required for binding of signal generating substances. A detailed discussion of suitable signal producing systems can be found in U.S. Patent No. 5,185,243, columns 11-13.

The label or other signal producing system members can be bound to a support or become bound to a molecule that is disposed on a support. Cells may be bound to a solid support in any manner known in the art, provided that the binding does not substantially interfere with the ability of the antibody to bind a marker on the cell. In some embodiments, the cells may be coated or covalently bound directly to the solid support. Linking groups may also be used to covalently couple the solid support and the cells. Other methods of binding the cells are also possible. For instance, a solid support may have a coating of a binder for a small molecule (such as, for example, avidin or an antibody) and a small molecule (such as, for example, biotin, hapten, etc.) can be bound to the cells or vice versa. The binding of components to the surface of a support may be direct or indirect, covalent or non-covalent and can be accomplished by well-known techniques, commonly available in the literature.

The support may be comprised of an organic or inorganic, solid or fluid, water insoluble material, which may be transparent or partially transparent. The support can have any of a number of shapes, such as particle, including bead, film, membrane, tube, well, strip, rod, planar surfaces (such as, e.g., sheet, plate and slide), and fiber, for example. Depending on the type of assay, the support may or may not be suspendable in the medium in which it is employed. Examples of suspendable supports are polymeric materials such as latex; lipid bilayers or liposomes; oil droplets, and metallic supports such as, e.g., magnetic particles; for example. Other support compositions include polymers, such as nitrocellulose, cellulose acetate, poly (vinyl chloride), polyacrylamide, polyacrylate, polyethylene, polypropylene, poly(4 methylbutene), polystyrene, polymethacrylate, poly(ethylene terephthalate), nylon, and poly(vinyl butyrate), either used by themselves or in conjunction with other materials.

The label and/or other signal producing system member may be bound to a member of a specific binding pair or another molecule. For example, the label can be bound covalently to a member of a specific binding pair such as, for example, an antibody, a receptor for an antibody, or a receptor that is capable of binding to a small molecule conjugated to an antibody. A receptor is a molecule capable of binding specifically to another molecule. Binding of the label to the specific binding pair member may be accomplished by chemical reactions that result in replacing a hydrogen atom of the label with a bond to the specific binding pair member or may include a linking group between the label and the specific binding pair member. Other signal producing system members may also be bound covalently to specific binding pair members. For example, two signal producing system members such as a fluorescer and quencher can each be bound to a different antibody that forms a specific complex with the coagulation factor. Formation of the complex brings the fluorescer and quencher in close proximity, thus permitting the quencher to interact with the fluorescer to produce a signal.

The assays discussed above are normally carried out in an aqueous buffered medium at a moderate pH, generally that which provides optimum assay sensitivity. The pH for the assay medium will usually be in the range of about 4 to about 11, or in the range of about 5 to about 10, or in the range of about 6.5 to about 9.5. The pH will usually be a compromise between optimum binding of the binding members of any specific binding pairs, and the pH optimum for other reagents of the assay such as members of a signal producing system, for example. Various buffers may be used to achieve the desired pH and maintain the pH during the incubation period. Illustrative buffers include borate, phosphate, carbonate, TRIS, barbital, PIPES, HEPES, MES, ACES, MOPS, BICINE, and the like. Various ancillary materials may be employed in the assay methods. For example, in addition to buffers and preservatives, the medium may comprise stabilizers for the medium and for the reagents employed. In some embodiments, in addition to these additives, proteins may be included, such as albumins; quaternary ammonium salts; polyanions such as dextran sulfate; and binding enhancers, for example. All of the above materials are present in a concentration or amount sufficient to achieve the desired effect or function.

In some embodiments, the one or more detectable labels comprise fluorescent tags. The fluorescent tags can be detected by a number of techniques known in the art including, but not limited to, direct immunofluorescence, FACS, immunoblotting, or any combination thereof.

The number of fluorescent tags used is dependent, in part, on the detection method employed and enables the CTCs to be distinguished from the normal cells.

In some embodiments, four fluorescent tags can be used. For example, the antibody that binds to vimentin, the antibody that binds to another marker expressed on circulating tumor cells, the antibody that binds to a marker expressed on white blood cells, and the antibody that binds to a marker expressed on endothelial cells can all have different fluorescent tags. When detected by immunofluorescence, the different fluorescent tags can be excited by different wavelengths of light such that each antibody is detected in a different channel.

In other embodiments, three fluorescent tags can be used. For example, the antibody that binds to vimentin and the antibody that binds to another marker expressed on circulating tumor cells can have a first fluorescent tag, the antibody that binds to a marker expressed on white blood cells can have a second fluorescent tag, and the antibody that binds to a marker expressed on endothelial cells can have a third fluorescent tag. When detected by immunofluorescence, the antibody that binds to vimentin and the antibody that binds to another marker expressed on circulating tumor cells can be detected in a first channel, the antibody that binds to a marker expressed on white blood cells can be detected in a second channel, and the antibody that binds to a marker expressed on endothelial cells can be detected in a third channel.

Alternatively, the antibody that binds to vimentin can have a first fluorescent tag, the antibody that binds to another marker expressed on circulating tumor cells can have a second fluorescent tag, and the antibody that binds to a marker expressed on white blood cells together with the antibody that binds to a marker expressed on endothelial cells can have a third fluorescent tag. When detected by immunofluorescence, the antibody that binds to vimentin can be detected in a first channel, the antibody that binds to another marker expressed on circulating tumor cells can be detected in a second channel, and the antibody that binds to a marker expressed on white blood cells together with the antibody that binds to a marker expressed on endothelial cells can be detected in a third channel.

In yet other embodiments, two fluorescent tags can be used. For example, the antibody that binds to vimentin and the antibody that binds to another marker expressed on circulating tumor cells can have a first fluorescent tag, and the antibody that binds to a marker expressed on white blood cells and the antibody that binds to a marker expressed on endothelial cells can have a second fluorescent tag. When detected by immunofluorescence, the antibody that binds to vimentin together with the antibody that binds to another marker expressed on circulating tumor cells can be detected in a first channel, and the antibody that binds to a marker expressed on white blood cells together with the antibody that binds to a marker expressed on endothelial cells can be detected in a second channel.

Signals can also be generated from the plurality of antibodies by indirect techniques. Indirect techniques do not require the plurality of antibodies to have a label. For example, in some embodiments, the detecting is indirect immunofluorescence comprising incubating the sample with a plurality of fluorescently labeled secondary antibodies. Thus, after the sample is incubated with a plurality of antibodies comprising an antibody that binds to vimentin, an antibody that binds to another marker expressed on CTCs, an antibody that binds to a marker expressed on WBCs, and an antibody that binds to a marker expressed on endothelial cells, the sample is further incubated with a plurality of fluorescently labeled secondary antibodies which bind to the plurality of antibodies.

The number of types of fluorescently labeled secondary antibodies used is dependent, in part, on the detection method and enables the CTCs to be distinguished from the normal cells. In some embodiments, the plurality of fluorescently labeled secondary antibodies can contain four types of fluorescently labeled secondary antibodies, such that the antibody that binds to vimentin can be bound by a first fluorescently labeled secondary antibody, the antibody that binds to another marker expressed on circulating tumor cells can be bound by a second fluorescently labeled secondary antibody, the antibody that binds to a marker expressed on white blood cells can be bound by a third fluorescently labeled secondary antibody, and the antibody that binds to a marker expressed on endothelial cells can be bound by a fourth fluorescently labeled secondary antibody. When detected by indirect immunofluorescence, the different fluorescent tags can be excited by different wavelengths of light such that each antibody is detected in a different channel.

In other embodiments, the plurality of fluorescently labeled secondary antibodies can contain three types of fluorescently labeled secondary antibodies. For example, the antibody that binds to vimentin and the antibody that binds to another marker expressed on circulating tumor cells can be bound by a first fluorescently labeled secondary antibody, the antibody that binds to a marker expressed on white blood cells can be bound by a second fluorescently labeled secondary antibody, and the antibody that binds to a marker expressed on endothelial cells can be bound by a third fluorescently labeled secondary antibody. When detected by immunofluorescence, the antibody that binds to vimentin and the antibody that binds to another marker expressed on circulating tumor cells can be detected in a first channel, the antibody that binds to a marker expressed on white blood cells can be detected in a second channel, and the antibody that binds to a marker expressed on endothelial cells can be detected in a third channel.

Alternatively, the antibody that binds to vimentin can be bound by a first fluorescently labeled secondary antibody, the antibody that binds to another marker expressed on circulating tumor cells can be bound by a second fluorescently labeled secondary antibody, and the antibody that binds to a marker expressed on white blood cells and the antibody that binds to a marker expressed on endothelial cells can be bound by a third fluorescently labeled secondary antibody. When detected by immunofluorescence, the antibody that binds to vimentin can be detected in a first channel, the antibody that binds to another marker expressed on circulating tumor cells can be detected in a second channel, and the antibody that binds to a marker expressed on white blood cells together with the antibody that binds to a marker expressed on endothelial cells can be detected in a third channel.

In yet other embodiments, the plurality of fluorescently labeled secondary antibodies can contain two types of fluorescently labeled secondary antibodies. For example, the antibody that binds to vimentin and the antibody that binds to another marker expressed on circulating tumor cells can be bound by a first fluorescently labeled secondary antibody, whereas the antibody that binds to a marker expressed on white blood cells and the antibody that binds to a marker expressed on endothelial cells can be bound by a second fluorescently labeled secondary antibody. When detected by immunofluorescence, the antibody that binds to vimentin together with the antibody that binds to another marker expressed on circulating tumor cells can be detected in a first channel, and the antibody that binds to a marker expressed on white blood cells together with the antibody that binds to a marker expressed on endothelial cells can be detected in a second channel.

In some embodiments, indirect immunofluorescence can be conducted using tyramide signal amplification (TSA) as described in the TSA kit manual from Perkin Elmer and Wang, G., Achim, C.L., Hamilton, R.L., Wiley, C.A., and Soontornniyomkij, V. Tyramide signal amplification method in multiple-label immunofluorescence confocal microscopy. Methods. 1999 Aug; 18(4):459-64. Briefly, TSA can be included in the CTC assay by incubating the filtered and fixed cells with a rabbit primary antibody to the target to be amplified plus other conjugated mouse antibodies. Next the slides can be incubated with an anti-rabbit HRP secondary antibody, followed by incubating the slides with tyramine-Alexa488 to perform the signal amplification.

Immunocytochemistry (ICC) can be employed to detect the plurality of antibodies, whether by direct or indirect immunofluorescence. For example, the patient sample can be placed on a solid support and treated to fix the cells and/or to permeabilize the cells, if desired. Fixation of the patient sample immobilizes the cells, preserving cell structure and maintaining the cells in a *in vivo*-like condition, which enables the cells to be recognized by a specific antibody. The amount of fixative employed is that which preserves the cells but does not lead to erroneous results in a subsequent assay. The amount of fixative depends on one or more of the nature of the fixative and the sample, for example. In some examples, the amount of fixative is about 0.05% to about 0.15% or about 0.05% to about 0.10%, or about 0.10% to about 0.15% by volume of the blood sample. Agents for carrying out fixation of CTCs include, but are not limited to, cross-linking agents such as an aldehyde reagent (e.g., formaldehyde, glutaraldehyde, and paraformaldehyde,); an alcohol (e.g., C1-C5 alcohols such as methanol, ethanol and isopropanol); a ketone (e.g., a C3-C5 ketone such as acetone). The designations C1-C5 or C3-C5 refer to the number of carbon atoms in the alcohol or ketone. One or more washing steps may be carried out on the fixed cells using a buffered aqueous medium.

If necessary after fixation, the sample may be subjected to permeabilization. In some instances, a fixation agent such as an alcohol (e.g., methanol or ethanol) or a ketone (e.g., acetone) also results in permeabilization and no additional permeabilization step is necessary. Permeabilization provides access through the cell membrane to markers of interest. The amount of permeabilization agent employed is that which disrupts the cell membrane and permits access to the marker. The amount of permeabilization agent depends on factors including the nature of the permeabilization agent. In some examples, the amount of permeabilization agent is about 0.1% to about 0.5%, or about 0.1% to about 0.4%, or about 0.1% to about 0.3%, or about 0.1% to about 0.2%, or about 0.2% to about 0.5%, or about 0.2% to about 0.4%, or about 0.2% to about 0.3%. Agents for carrying out permeabilization of the sample includes, but is not limited to, an alcohol (e.g., C1-C5 alcohols such as methanol and ethanol); a ketone (e.g., a C3-C5 ketone such as acetone); a detergent (e.g., saponin, TRITON® X-100, and TWEEN®-20). One or more washing steps may be carried out on the permeabilized cells using a buffered aqueous medium.

Following fixation and permeabilization, the sample can be contacted with an aqueous medium containing the plurality of antibodies. The aqueous medium may be an assay medium as described above and the amount of each labeled antibody is that which is sufficient to identify each of the markers of interest. In some examples, the amount of each labeled antibody can be in excess of the suspected amount of marker. The cells can be incubated with the labeled antibodies under conditions that permit binding of the labeled antibody to their respective markers. Such conditions are discussed above regarding assays in general. After incubation, the sample can be subjected to one or more washing steps using an aqueous buffered medium to remove unbound labeled antibodies.

A fluorescent DNA stain such as, for example, 4',6-diamidino-2-phenylindole, propidium iodide, ethidium bromide, SYBR® Green I, VISTRA™ GREEN, SYTO® GREEN, SYBR® Gold, YO-PRO-1™, TOTO-3™, TO-PRO-3™, NUCLEAR-ID™ Red, or Hoechst dye, can be employed to enhance contrast in the sample image during microscopic examination. After staining, one or more washing steps may be carried out on the cells using a buffered aqueous medium. The cells can then be examined using a fluorescent microscope and each of the different fluorescent labels can be used in the direct detection of a respective marker in the sample.

Alternatively, antibodies may be employed where the respective antibodies are indirectly detected with a second antibody, which is labeled with an enzyme, or directly labeled with an enzyme in an enzyme amplification method. A respective enzyme such as, for example, horseradish peroxidase (HRP) can be detected by appropriate means. For example by enzymatic generation of a respective fluorescent, optical, or chemiluminescent signal, a respective enzyme label can be generated to a covalently attached dye to protein tyrosine groups in the presence of HRP (such as Tyramide Signal Amplification (TSA™), Perkin Elmer). The specific binding members may be, for example, an antibody specific for each of the respective unlabeled antibodies used for binding to a respective marker.

The cells can then be examined using a fluorescent microscope and each of the different fluorescent labels can be used in the direct detection of a respective markers.

Screening for false positives in the sample comprises identifying single cells that have a signal from the antibody that binds to vimentin, a signal from the antibody that binds to the another marker expressed on circulating tumor cells, or both, together with a signal from the antibody that binds to the marker expressed on endothelial cells. In some embodiments, screening for false positives in the sample comprises identifying single cells that have a signal from the antibody that binds to vimentin together with a signal from the antibody that binds to the marker expressed on endothelial cells. In some embodiments, screening for false positives in the sample comprises identifying single cells that have a signal from the antibody that binds to the another marker expressed on CTCs together with a signal from the antibody that binds to the marker expressed on endothelial cells. In yet other embodiments, screening for false positives in the sample comprises identifying single cells that have a signal from the antibody that binds to vimentin, a signal from the antibody that binds to the another marker expressed on CTCs together with a signal from the antibody that binds to the marker expressed on endothelial cells.

Confirming the presence of a circulating tumor cell in the sample comprises identifying one or more cells that have a signal from the antibody that binds to vimentin and a signal from the antibody that binds to the another marker expressed on CTC, but not a signal from the antibody that binds to the marker expressed on white blood cells, a signal from the antibody that binds to the marker expressed on endothelial cells, or both.

When using direct or indirect immunofluorescence, false positives or CTCs can be identified by overlaying/merging the signals from the various channels used (2, 3, or 4 dependent upon the number of fluorescent tags). For example, a cell that fluoresces in the channel used to detect the antibody that binds to vimentin and the channel used to detect the antibody that binds to another marker expressed on CTCs but not in the channel used to detect the antibody that binds to a marker expressed on endothelial cells is a CTC. This represents a signal specific to the CTCs. On the other hand, a cell that fluoresces in the channel used to detect the antibody that binds to vimentin, in the channel used to detect the antibody that binds to another marker expressed on CTCs, as well as the channel used to detect the antibody that binds to a marker expressed on endothelial cells is a false positive, i.e. a normal cell.

The amount of signal obtained in any of the methods provided herein can be compared to a threshold to evaluate the accuracy of the results. As used herein, the term "threshold" means the point at which a signal is detectable over a noise, wherein the noise is the background signal from the detecting technique. For example, in some embodiments, the signal from the antibody that binds to vimentin and the signal from the antibody that binds to another marker on CTCs are preferably above a threshold. In other embodiments the signal from the antibody that binds to a marker on WBCs and the signal from the antibody that binds to a marker on endothelial cells are preferably below a threshold. The WBC and endothelial cell signal thresholds can be established using a negative control with WBCs and endothelial cells, respectively. The CTC signal threshold can be determined using a positive control, including WBCs and/or cultured endothelial cells that are fixed with a CTC marker. Signals below the threshold can be set to zero. The thresholds are specific to the quality of degree of separation of bound from unbound labels, and dependent on the method of enrichment used.

Control cells include those of endothelium origin including, but not limited to, Human Umbilical Vein Endothelial Cells (HUVEC), Human Large Vessel Endothelial Cells (HLVEC), and Human Small Vessel Endothelial Cells (HSVEC), for example, listed in the ATCC® and other cell bank collection of primary and transformed endothelial cell lines from a variety of species. Cancer cells of epithelium origin include, but are not limited to, breast carcinoma cells (SKBR-3, SKBR-5, MDA, Hs 849.T, HTB30), non-small cell lung adenocarcinoma (N2228), brain astrocytoma (CCF-STTG1, SW 1783), cervix adenocarcinoma (HeLa), colon adenocarcinoma (H329, GH354, COLO 824), pancreatic adenocarcinoma (KLE, HPAC), renal cell adenocarcinoma (786-O, 769-P, MIA), prostate carcinoma cells (MDA PCa, LNCaP), pancreatic carcinoma cells (PaCa-2), bladder carcinoma (HT-1376, Hs 228.T), colorectal carcinoma (SNU-C2B, LS411N), hepatocellular carcinoma (SNU-449, C3A), pharynx squamous cell carcinoma (FaDu), transitional bladder carcinoma cells (SW 7801), melanoma (CHL-1), and others listed in the ATCC® and other cell bank collections. ATCC® has approximately 1,100 tumor cell lines from a variety of species. Cancer cells of mesenchymal origin include, but are not limited to, fibrosarcoma connective tissue (15.T), lymphosarcoma lymph node (TE 175.T), adenocarcinoma, non-small cell lung cancer (H226), osteosarcoma (143.98.2), along with other stromal, stem cell, and sarcoma, and others listed in the ATCC® and other cell bank collections. Cells of blood origin include those from healthy donor blood, as well as patient blood including, but not limited, leukemia bone marrow, myeloblast (KG-1), acute monocytic leukemia (THP-1), acute T cell leukemia (J.CaM1.6), acute promyelocytic leukemia (15 HL-60), lymphoma (1A2), and others listed in the ATCC® and other cell bank collections of cell lines of blood origin.

In some embodiments, the disclosed methods further comprise correlating the signal specific to the circulating tumor cells with a tumor burden. As used herein, the term "tumor burden" refers to the number of cancer cells, size of the tumor, or the amount of cancer in the patient. For example, after evaluating or confirming the presence of CTCs, the CTCs can be counted when the signal from the antibody that binds to vimentin and the signal from the antibody that binds to another marker on CTCs are above a threshold, and the signal from the antibody that binds to a marker on WBCs and the signal from the antibody that binds to a marker on endothelial cells are below a threshold.

Prior to incubating the sample with a plurality of antibodies, the patient sample can be treated to enrich the concentration of circulating tumor cells. Enrichment of the concentration of CTCs in a sample from a patient may be achieved by any number of methods known in the art including, but not limited to, filtration. Filtration can be achieved by using one or more of the following forces: centrifugation, pressure, vacuum, capillary hydrostatic forces, ultracentrifugation, differential thermal gradient and electrophoretic forces, for example. Filtration involves contacting the sample with a porous matrix usually under vacuum pressure. In some examples, the porous matrix may be part of a filtration module where the porous matrix is part of an assembly for convenient use during filtration ("porous matrix filtration assembly"). The porous matrix can be a membrane, film, or microfluidic structure that has pores or obstacles through which liquid with cells flow across or through. Filtration techniques include, but are not limited to, microfiltration, ultrafiltration, cross-flow filtration, or any combination thereof.

One example of a porous matrix filtration assembly, by way of illustration and not limitation, is described by Friedrich, et al., in U.S. Patent Application Publication No. 2012/0315664 ("'664 patent application publication). The '664 patent application publication discloses an assembly and method for the filtration of a liquid. A supporting body is designed in a recess of a carrier and a filter membrane lies flat on the supporting body. The filter membrane and the supporting body are designed to be permeable to liquids and thus serve as filters, in particular for filtering rare cells from blood. As a result of the filter membrane lying level on the supporting body, the filtration residue can be particularly well examined microscopically.

The container in which the patient's sample is collected can contain a collection medium into which the sample is delivered. The collection medium can comprise a dry medium and may comprise an amount of platelet deactivation agent effective to achieve deactivation of platelets in a blood sample. The collection medium may also comprise other agents including, but not limited to, an anti-coagulant, a fixing agent, a fibrin-formation-arresting agent, or any combination thereof, each of which are present in an amount sufficient to achieve the desired function of the agents. In some examples, a sample may be treated to preferentially agglutinate CTCs over normal cells to facilitate separation of CTCs from normal cells, for example, during filtration.

Platelet deactivation agents include, but are not limited to, chelators, citrate, cyclooxygenase inhibitors, adenosine diphosphate (ADP) receptor inhibitor, glycoprotein IIB/IIIA inhibitors, phosphodiesterase inhibitors, thromboxane inhibitors, and adenosine reuptake inhibitors. Chelating agents include chelating agents that comprise a triacetic acid moiety or a salt thereof, a tetraacetic acid moiety or a salt thereof, a pentaacetic acid moiety or a salt thereof, or a hexaacetic acid moiety or a salt thereof. In some examples, the chelating agent is ethylene diamine tetraacetic acid (EDTA) and its salts or ethylene glycol tetraacetate (EGTA) and its salts. The effective amount of platelet deactivation agent is dependent upon such factors as the nature of the platelet deactivation agent, the nature of the blood sample, level of platelet activation and ionic strength. In some examples, the amount of dry EDTA in the container is that which will produce a concentration of about 1.0 mg/mL to about 2.0 mg/mL of blood, or about 1.5 mg/mL of the blood. The amount of the platelet deactivation agent is that which is sufficient to achieve at least about 90%, or at least about 95%, or at least about 99% of platelet deactivation.

A fibrin-formation-arresting agent is a substance that, when combined with the blood sample, stops the formation of fibrin on the surface of the CTCs, which forms as a result of naturally occurring clotting proteins in the blood sample such as, for example, fibrinogen. Such fibrin-formation-arresting agents include, but are not limited to, platelet deactivation agents, anti-coagulants and fixing agents.

Anti-coagulants include, but are not limited to, Factor VII inhibitors, Factor X inhibitors, direct thrombin inhibitors, coumarins, heparin, and anti-thrombin proteins.

Fixing agents include, but are not limited to, substances that act to cross-link proteins and/or to disable proteolytic enzymes and prevent natural generation of fibrin. In some examples, the fixing agent is an aldehyde reagent (such as, e.g., formaldehyde, glutaraldehyde, and paraformaldehyde) and ureas (such as, e.g., diazolidinyl urea or imidazolidinyl urea).

The porous matrix can be a solid or semi-solid material and can be comprised of an organic or inorganic, water insoluble material. The porous matrix can have any of a number of shapes including, tubular (e.g., hollow fiber, spiral wound, and hollow fine fiber), track-etched, or planar or flat surface (e.g., strip, disk, film, membrane, and plate). The matrix may be fabricated from a wide variety of materials, which may be naturally occurring or synthetic, polymeric or non-polymeric, fibrous or non-fibrous, including, but not limited to, cellulose (including paper), nitrocellulose, cellulose acetate, polycarbonate, poly (vinyl chloride), polyacrylamide, polyacrylate, polyethylene, polypropylene, poly-(4 methylbutene), polystyrene, polymethacrylate, poly(ethylene terephthalate), nylon, and poly(vinyl butyrate), ceramic material, metallic material, or any combination thereof.

The size of the pores of the porous matrix is that which is sufficient to preferentially retain CTCs while allowing the passage of normal cells through the pores. The size of the pores of the porous matrix is dependent on, for example, the size of the CTC and the normal cells, and the pressure applied to the sample such as a blood sample. In some examples the average size of the pores of the porous matrix is about 1 µm to about 100 µm, or about 1 µm to about 75 µm, or about 1 µm to about 50 µm, or about 1 µm to about 20 µm, or about 1 µm to about 10 µm, or about 5 µm to about 100 µm, or about 5 µm to about 75 µm, or about 5 µm to about 50 µm, or about 5 µm to about 20 µm, or about 5 µm to about 10 µm, for example. The density of pores in the porous matrix is about 1% to about 80%, or about 10% to about 80%, or about 20% to about 80%, or about 50% to about 80%, or about 20% to about 70%, for example.

Pressure can be applied to the sample on the porous matrix to facilitate passage of normal cells through the membrane. The term "pressure" refers to pressure differences from normal atmospheric pressure and can be either positive pressure (increase in pressure relative to normal atmospheric pressure) or negative pressure (vacuum) (decrease in pressure relative to normal atmospheric pressure). The level of pressure applied is dependent, for example, on one or more of the nature and size of the non-rare cells, the size of the CTC, the nature of the porous matrix, and the size of the pores of the porous matrix. In some examples, the level of positive pressure applied is about 1 millibar to about 500 millibar, or about 1 millibar to about 400 millibar, or about 1 millibar to about 300 millibar, or about 1 millibar to about 200 millibar, or about 1 millibar to about 100 millibar, or about 1 millibar to about 50 millibar, or about 1 millibar to about 30 millibar, or about 1 millibar to about 25 millibar, or about 1 millibar to about 20 millibar, or about 1 millibar to about 15 millibar, or about 1 millibar to about 10 millibar, or about 5 millibar to about 30 millibar, or about 5 millibar to about 25 millibar, or about 5 millibar to about 20 millibar, or about 5 millibar to about 15 millibar, or about 5 millibar to about 10 millibar, for example. The level of negative pressure (vacuum) applied is the negative of the above ranges.

In some embodiments, the pressure applied to the sample on the porous matrix can be an oscillating pressure. "Oscillating pressure" refers to a pressure applied intermittently at regular or irregular intervals, which may be, for example, about 1 second to about 600 seconds, or about 1 second to about 500 seconds, or about 1 second to about 250 seconds, or about 1 second to about 100 seconds, or about 1 second to about 50 seconds, or about 10 seconds to about 600 seconds, or about 10 seconds to about 500 seconds, or about 10 seconds to about 250 seconds, or about 10 seconds to about 100 seconds, or about 10 seconds to about 50 seconds, or about 100 seconds to about 600 seconds, or about 100 seconds to about 500 seconds, or about 100 seconds to about 250 seconds, for example. In this approach, pressure can be oscillated at about 0 millibar to about 10 millibar, or about 1 millibar to about 10 millibar, or about 1 millibar to about 7.5 millibar, or about 1 millibar to about 5.0 millibar, or about 1 millibar to about 2.5 millibar, for example, during some or all of the application of pressure to the blood sample. Oscillating pressure is achieved using an on-off switch, for example, and may be conducted automatically or manually. High pressure drops are allowable depending on one or more of reservoir volume, sample volume and filtration rate.

The sample can be contacted with a porous matrix such that CTCs are preferentially retained on the porous matrix and normal cells pass through the porous matrix. In some embodiments, the sample can be diluted with a dilution medium prior to contact with the porous matrix. The dilution medium can comprise an aqueous medium, which may be buffered. The pH for an aqueous buffered medium is usually a moderate pH. In some examples the pH of the dilution medium is about 5 to about 8, or about 6 to about 8, or about 7 to about 8, or about 5 to about 7, or about 6 to about 7, or physiological pH, for example. Various buffers may be used to achieve the desired pH and maintain the pH during any incubation period. Illustrative buffers include, but are not limited to, borate, phosphate (e.g., phosphate buffered saline), carbonate, TRIS, barbital, PIPES, HEPES, MES, ACES, MOPS, and BICINE. The amount of dilution medium combined with the sample is dependent on one or more of a number of factors such as, for example, the nature of the porous matrix and the nature of the sample. In some embodiments, the amount of dilution medium is about 5 mL to about 100 mL, or about 5 mL to about 80 mL, or about 5 mL to about 60 mL, or about 5 mL to about 50 mL, or about 5 mL to about 30 mL, or about 5 mL to about 20 mL, or about 5 mL to about 10 mL, or about 10 mL to about 100 mL, or about 10 mL to about 80 mL, or about 10 mL to about 60 mL, or about 10 mL to about 50 mL, or about 10 mL to about 30 mL, or about 10 mL to about 20 mL, or about 20 mL to about 100 mL, or about 20 mL to about 80 mL, or about 20 mL to about 60 mL, or about 20 mL to about 50 mL, or about 20 mL to about 30 mL, for example, based on 10 mL of the sample.

The sample can be contacted with the porous matrix for a period of time sufficient to achieve an enrichment of the CTCs to normal cells on the porous matrix. The period of time is dependent on, for example, one or more of the nature and size of the normal cells, the size of the CTCs, the nature of the porous matrix, the size of the pores of the porous matrix, the level of pressure applied to the blood sample on the porous matrix, the volume to be filtered, and the surface area of the filter. In some examples, the period of contact is about 1 minute to about 1 hour, about 5 minutes to about 1 hour, or about 5 minutes to about 45 minutes, or about 5 minutes to about 30 minutes, or about 5 minutes to about 20 minutes, or about 5 minutes to about 10 minutes, or about 10 minutes to about 1 hour, or about 10 minutes to about 45 minutes, or about 10 minutes to about 30 minutes, or about 10 minutes to about 20 minutes, for example.

The desired increase in the ratio of CTCs to normal cells in the sample can be at least about 10 fold, or at least about 20 fold, or at least about 50 fold, or at least about 75 fold, or at least about 100 fold, or at least about 200 fold compared to the original sample. In some examples, the increase of the ratio of CTC to normal cells that is desired is about 10 fold to about 200 fold, or about 10 fold to about 150 fold, or about 10 fold to about 100 fold, or about 25 fold to about 100 fold, or about 50 fold to about 100 fold, for example.

In yet other embodiments, the disclosed methods further comprise isolating the circulating tumor cells from the sample. For example, after the presence of CTCs is confirmed, the CTCs can be isolated from the sample for, for example, further analysis or treatment testing. Numerous techniques can be used to isolate the CTCs from the sample, including, but not limited to, magnetic particles, filtration, flow cytometry, microfluidic channels and chambers, or any combination thereof.

Any of the methods of screening a patient sample in a CTC assay and methods of performing CTC detection assays described herein can be used for screening for false positives in the disclosed treatment methods.

### EXAMPLES

### Materials

All chemicals may be purchased from the Sigma-Aldrich Company (St. Louis MO) unless otherwise noted.

### Abbreviations

The following abbreviations are used herein: mL = milliliter; µL = microliter; mg = milligram; µm = micron(s); min = minute(s); h = hour(s); rpm = revolutions per minute; PBS = phosphate buffered saline as Ambion PBS pH 7.4 Thermo Scientific product number 158-0020; K₃EDTA = potassium salt of ethylenediaminetetraacetate; ATCC = American Type Culture Collection.

### Collection of samples from healthy persons and cancer patients

Whole blood specimens for testing were prepared by collection of blood from normal donors lacking any rare/diseased cells (i.e. cancer cells) and from disease donors with metastatic cancer and circulating tumor cells. The blood samples (7-10 mL) were collected into a Blood Collection Tubes containing K₃EDTA and 0.45 ml Transfix (Vacutest Kima TVT-09-50-45). WBC were about 10⁷ per 10 mL blood and RBC were about 5x10¹⁰ per 10 mL of blood.

### Preparation of cell controls

Cells were grown in culture using the recommended growth media. Human breast cancer cell line SK-BR-3 (ATCC® HTB-30) was grown in IMDM Modified (HYCLONE®) (Pierce/Thermo Scientific, Rockford IL) plus 15% FBS (HYCLONE®). Human lung cancer cell line NCI H226 (ATCC® CRL-5826) was grown in 10% FBS + RPMI-1640 (HYCLONE®). Cell line primary human umbilical vein endothelial cells (HUVEC) (ATCC® PCS-100-010) were grown in ENDOGRO® Basal Media (Millipore Corporation, Billerica MA). All of the following operations were carried out under strict aseptic conditions. Cells in 6 mL complete medium were dispensed into 75 cm² culture flasks, each containing 3 mL of complete medium. The culture was incubated at 37°C in a suitable incubator with 5% CO₂ in air atmosphere. Cells were sub-cultured two or three times weekly. When the culture was confluent, culture medium was removed, the cell layer was washed with 5 mL of 1 x PBS and treated with trypsin, and cell cultures were split.

Cultured cells were counted by mixing 30 uL of trypan blue solution (0.4%) and 10 uL of cells and reading on a hemocytometer or by reading cell suspension undiluted. Typically cells at 10⁵/ mL were transferred to empty and clean blood sample tubes and washed down with 10 mL of whole blood from healthy donors collected in a Blood Collection Tubes containing K₃EDTA and 0.45 ml Transfix (Vacutest Kima TVT-09-50-45). Controls were made with 0, 5, and at additional levels between 20 to 300 cells/ blood tube. Expected values were assigned to these samples for use in measuring recovery of cells.

### Filtration and staining of patient samples and control samples

Within 5 days after storage at 25°C, the blood samples were filtered through a membrane having an average pore size of 8 µm according to a method disclosed in U.S. Patent Application Publication No. 2012/0315664. During filtration, the sample on the membrane was subjected to a negative mBar, that is, a decrease greater than about -30 mBar from atmospheric pressure. The vacuum applied varied from 1 to -30 mBar as the volume of the sample reduces during filtration. High pressure drops were allowable dependent on reservoir and sample volume and filtration rate. Just prior to filtration, a sample (7-10 mL) was transferred to a 50 mL Falcon tube, which was filled to 20 mL with cold PBS with 0.2 to 10 mg/L Fibrin added. The Falcon tubes were manually overturned twice and subjected to centrifugation for 10 min. at 400 x g at 20°C. The diluted sample was placed into the filtration station without mixing and the diluted sample was filtered through the membrane. Following the filtration, the membrane was washed with PBS, and the sample was fixed with formaldehyde, washed with PBS, subjected to permeabilization using of 0.2% Triton® X100 in PBS, and washed again with PBS.

Cells captured on the membrane were detected with an immunocytochemistry (ICC) procedure based on the binding of specific antibodies to specific proteins or antigens in cells. A blocking buffer of 10% casein in PBS was dispensed on the membrane. After an incubation period of 5 min, the membrane was washed with PBS to block non-specific binding to the membrane. Next, an antibody-conjugate mix was dispensed to the membrane followed by an incubation period of 20 min at RT. The mixture of antibody conjugates (in 10% casein in PBS) used in this example included anti-cancer cell antibodies (reactive to Vimentin, CK8/18 and 19) conjugated to DyLight 550 (Thermo Scientific Inc. Rockford, IL) at 15 µg/mL, and anti-CD45 antibody (used for WBC) conjugated to DyLight 650 at 20 µg/mL. The mixture of antibody conjugates also included anti-endothelial cell antibody (reactive to CD144, ZO1 or CD105) conjugated to DyLight 488 at 15 µg /mL. Unbound antibody was washed away (PBS + 0.05% Tween® 20) and DAPI (0.8 µg/mL in PBS), a fluorescent DNA stain, was added to stain the nuclei of the cells. A last wash step with PBS was performed, followed by cover media to help preserve the fluorescent intensity of the probes. Slides were made with DABCO as a cover slip medium (0.25 g DABCO to 9 mL glycerol and 1 ml 10 x PBS).

### Methodology for measurement

Slides were placed on a slide holder of a fluorescent microscope (Leica DM5000 (Leica Microsystems GmbH, Wetzlar, Germany)) where images were captured during the automated scanning of the membrane for each of the fluorescent probes used for detection of targeted cells. Antibody-conjugates became bound to the protein or antigen of a cell, and the fluorescent labels were detected by using a fluorescent microscope with excitation, emission, and cut-off filters specific for each label. Cells were then characterized by scanning the membrane by fluorescence microscopics conducted with a Leica DM5000 using the filter sets for respective fluorophore labels used in the antibody-conjugates above (namely, DyLight 480, DyLight 550, and DyLight 650, ThermoFisher Scientific, Inc., Waltham MA), and DAPI. The multiple fluorescent labels, each with different cell type specific antibodies, were used to detect multiple antigens or proteins in the isolated cells. The filtration process allows all signals to be measured simultaneously. The measurement of an amount of signal obtained in any of the cell types were correlated with the level of disease in the subject. This measurement is explained more fully as follows: To first determine if the cell is of interest using a CTC marker with separate distinct signal and by comparison to an non-CTC marker in a second or third separate distinct signal. The CTCs are counted when the CTC signal is above a threshold and the non-CTC signal is below a threshold for the CTC labels. The non-CTCs are counted when the non-CTC signal is above a threshold and the CTC signal is below a threshold for the non-CTC labels. The threshold is defined as the point at which the signal is detectable over the noise (i.e. the background signal on the filtration matrix). These thresholds are set using known cell types as control cells, including Human Umbilical Vein Endothelial Cells (HUVEC), those of endothelium, cancer cells of epithelium origin including breast carcinoma cells (SKBR-3), and non-small cell lung adenocarcinoma (H226), cancer cells of mesenchymal origin. These cells were compared to normal cells of blood origin those from healthy donor blood.

A separate signal threshold was selected for each determination of whether a cell is a CTC, a non-CTC or whether a CTC has additional circulating endothelial cell features. The non-CTC signal threshold is established using a negative control from a health patient with non-CTCs and circulating endothelial cells. The CTC and additional feature signal thresholds are determined using a positive control with non-CTC and cultured CTCs that are fixed with the additional feature. Signals below the threshold are set to zero. The unknown sample is run and only the additional feature signals above the threshold measured on cells that are CTC positive and non-CTC negative cells is considered a positive indicator of a cell having an additional feature.

### Recovery of CTCs using filtration

FIG 1 represents a line graph showing CTC recovery using fixed cancer cells from tissue culture spiked into blood from healthy donors. Blood samples from cancer patients were filtered and analyzed using antibodies to vimentin (red) and CK8/18/19 (red) as cancer cell markers, and antibodies to CD45 (purple) as a white blood cell marker. DAPI was used to stain the cellular nuclei. (FIG 2). As shown from FIG 2A, 2B, 2C and 2D, the samples from cancer patients contained cells that stained red, suggesting the presence of cancer cells following the filtration procedure. The recovery of cancer cells was evaluated by analyzing blood samples from healthy donors spiked with known numbers of cancer cells from tissue culture. The samples were filtered and the number of cancer cells in each sample was counted. Staining was examined as done in FIG 2. Enrichment of CTCs was measured by counting the CTCs (either CK or vimentin positive) and comparing to the count of the normal WBC (CD45 positive) remaining on the membrane. RBCs were either lysed or passed through the membrane; all CTCs were captured on the membrane. FIG. 1 shows a linear recovery of CTCs at > 90%.

### Evaluation of cancer cell markers

To identify appropriate cancer antibodies in the CTC assay, control cells for particular cancer types were screened against antibodies reactive to cancer markers and labeled with Dylight 550. Cytokeratins 8/18/19 antibodies were reactive to carcinoma cell lines, breast carcinoma cells (SKBR), and non-small cell lung adenocarcinoma (H226). The vimentin antibodies were reactive to non-small cell lung adenocarcinoma (H 226) and SKBR. The signals obtained with the control cells allowed determining the cancer cell thresholds by comparison to normal WBC in the samples. (Data not shown)

### Evaluation of endothelial markers to screen for false positives

To identify appropriate markers that can be used for the identification of circulating endothelial cells (CECs) in a CTC assay, blood from healthy donors (FIG 3A and 3C) and blood from healthy donors spiked with HUVEC cells (FIG 3B and 3D) were screened using a variety of antibodies to endothelial cell markers (Table 1). The samples were analyzed with antibodies to vimentin (red) and CK8/18/19 (red) as cancer cell markers, antibodies to CD45 (purple) as a white blood cell marker, and antibodies to various endothelial cell markers. DAPI (blue) was used to stain cellular nulei. FIG 3, comprising FIGS 3A, 3B, 3C and 3D represents an overlay of immunofluorescence images showing CECs found in the blood samples. Spiked in HUVEC cells are shown in a cluster for blood samples from healthy donors in 3B and 3D. Of the markers screened, CD105 had the strongest response to endothelial control cells (HUVEC). Additional staining of cells was conducted by H&E staining to identify potential naturally circulating endothelial cells in the normal patient samples (FIG 4).

**Table 1**

| **Marker** | **Antibody Clone** | **CEC naturally in health patient blood** | **Cultured HUVECs added to health patient blood** |
|---|---|---|---|
| Vimentin | V9 | ++++ | + * |
| ZO-1 | ZO1-1A12 | + | + |
| CD105 | MEM-226 | + | ++++ |
| CD326/EpCAM | B29.1 (VU1D9) | - | - |
| CD144 | F-8 | +++ | + |
| | BV9 | - | - |
| | 123413 | - | - |
| | 123433 | - | + |
| | TEA 1/31 | - | ++ |
| | MM0012-8A0 | - | - |
| Claudin 5 | 4C3C2 | - | |
| ESAM | 408159 | - | + |
| VEGFR2 | Avia 12alpha1 | - | - |
| von Willebrand Factor | EPSISR15 | - | +++ |
| | 210905 | - | - |
| | 2Q2134 | - | + |
| | MM0601-5F2 | - | - |
| | RFF-VIII R/1 | - | - |

| | | | |
|---|---|---|---|
| * Variable and weak response to VIM. ** One single cell observed with response to claudin 5 but not CD45 or CK/vim | | | |

Surprisingly CECs were also detected by vimentin in normal health patients. FIG 3A and 3C represents an overlay of immunofluorescence images showing false positive cells found in blood samples from healthy donors without spiked in HUVEC. The samples were analyzed with antibodies to vimentin (red) and CK8/18/19 (red) as cancer cell markers, and antibodies to CD45 (purple) as a white blood cell marker. DAPI (blue) was used to stain cellular nulei. These cells were additionally stained with H&E to showing they were not WBC. FIG 4, comprising FIGS 4A, 4B, 4C and 4D represents immunocytochemistry (ICC) using anti-vimentin antibodies and hematoxylin and eosin (H&E) staining of (A) and (C) filtered cancer cells and (B) and (D) false positives. (A) and (C) Vimentin (top left), DAPI (top right), overlay of vimentin and DAPI (bottom left) and H&E staining (bottom right). (B) and (D) Vimentin (top left), CD45 (top right), vimentin and DAPI (bottom left) and H&E staining (bottom right).

These health donor samples were screened with markers for identification of endothelial cells (Table 1). Samples were incubated with anti-vimentin monoclonal antibodies (red), anti-CD45 monoclonal antibodies (purple), and anti-endothelial (green) (anti-ZO-1 in FIG 5A and 5C and anti-CD144 in FIG 5B and 5D) monoclonal antibodies. CK8/18/19 was not used to ensure that the signal was specific to vimentin. The response to CD144 and ZO1 indicate that the cells are CECs. The cells were also stained with DAPI. As shown in FIGS 5A, 5B, 5C and 5D upon overlaying the images, the red, purple and green signals overlapped, showed the vimentin false positive cells react to endothelial markers. This data indicates the vimentin would react to both CTC and CEC in the sample.

Also unexpected was the level of response from most endothelial cells. The response from CEC in health patient blood was weaker than the response from cultured HUVEC. FIG 6 illustrates that CD105 detects HUVEC but not native CEC, whereas vimentin detects native CTC but not HUVEC. Unexpectedly, the cancer cell marker vimentin was strongly positive for natural CEC. In contrast, the vimentin V9 antibody and CD144 F-8 antibody responded more strongly to the CEC in health patient blood than to the HUVEC cell. This data also shows that the CD144 would specifically measure CECs, as CTCs were not positive for CD144. The signals obtained with the control cells allow determination of the non-CTC thresholds by comparing the WBC to the CTCs in the samples. Adjustments to thresholds were made to account for the over or under estimation of the control cells to native cells such that native CEC would be detected and WBC would not.

The above methods may be employed to determine the potential of a mammalian subject for exhibiting disease. The signal obtained in any of the above assay methods for CTC detection comprising one or more additional features can be correlated to the potential of a mammalian subject for exhibiting disease by measuring the CTC and non-CTC signals identifying the cells above the CTC threshold and below the non-CTC threshold. The additional feature signal on the CTC can then be measured if above the positive threshold. The numbers of CTC can be counted for each patient. If the signal for the feature is above the threshold for the feature, the cell is considered to have this feature. The higher the increase in the feature signal above the threshold, the greater is the feature for the CTC. The higher the number of CTCs, the number of CTCs with features and the extent of additional features, the greater the correlation of the subject exhibiting disease versus subjects lacking disease.

### Identification of false positives

Blood from 9 different health donors was incubated with anti-CK8/18/19 (red) antibodies, anti CD-144 antibodies (green), and anti CD-45 antibodies (purple) with or without anti-vimentin antibodies (red), and evaluated by microscopy. Single cells as well as doublets and clusters were observed from these samples. As shown in Table 2, only the cells incubated with anti-vimentin antibody appeared in the red channel, suggesting that anti-vimentin antibody is the source of the false positive. While not wishing to be bound to theory, it is believed that cells of endothelium origin surprisingly cause false positives with vimenten, which can be ruled out with markers specific for cells of endothelium origin (namely CD 144 in this example) and cells of blood origin (namely CD45 in this example).

**Table 2**

| **Sample #** | **Donor #** | **# positive cells in red channel** | | |
|---|---|---|---|---|
| | | **CK8/18/19 or vimentin positive** | **CK8/18/19 positive** | **CK8/18/19 or vimentin positive and CD144 negative** |
| 1 | 171 | 2 | 0 | 0 |
| 2 | 128 | 8 | 0 | 0 |
| 3 | 77 | 3 | 0 | 0 |
| 4 | 155 | 2 | 0 | 0 |
| 5 | 189 | 2 | 0 | 0 |
| 6 | 153 | 4 | 0 | 0 |
| 7 | 99 | 4 | 0 | 0 |
| 8 | 55 | 1 | 0 | 0 |
| 9 | 73 | 0 | 0 | 0 |
| 10 | 73 (2^{nd} tube) | 2 | 0 | 0 |

### Identification of true CTC positives

Blood from 4 different breast cancer patients and 6 different lung cancer patients were incubated with anti-CK8/18/19 antibodies (red), anti-CD-144 antibodies (green), and anti-CD-45 antibodies (purple), with or without anti-vimentin antibodies (red), and evaluated by microscopy. Single cells as well as doublets and clusters were observed from these samples. As shown in Table 3, only the samples incubated with anti-vimentin antibody had significantly more cells identified as positive, even with the elimination of false positive CEC using CD144 response. The inclusion of vimentin is shown in increased detection rate for cancer cells, as vimentin appears to detect cells of epithelium origin and cells of mesenchymal origin. Additionally, the simultaneous detection of CTC and CEC can be achieved; a cell that is CK8/18/19 or vimentin positive and CD144 negative cell is a CTC, whereas a cell that is CK8/18/19 or vimentin positive and CD144 positive cell is a CEC.

**Table 3**

| **Sample #** | **Cancer** | **# positive cells in red channel** | | |
|---|---|---|---|---|
| | | **CK8/18/19 or vimentin positive** | **CK8/18/19 positive** | **CK8/18/19 or vimentin positive and CD144 negative** |
| 1 | breast | 15 | 1 | 2 |
| 2 | breast | 8 | 4 | 8 |
| 3 | breast | 1 | 1 | 1 |
| 4 | breast | 10 | 9 | 10 |
| 5 | lung | 9 | 0 | 7 |
| 6 | lung | 1 | 1 | 1 |
| 7 | lung | 5 | 4 | 5 |
| 8 | lung | 59 | 1 | 14 |
| 9 | lung | 6 | 6 | 6 |
| 10 | lung | 6 | 0 | 5 |

### CTC indirect amplification method

Given that direct immunostaining in the CTC assay requires antibodies with high affinities to generate sufficient signal, tyramide signal amplification (TSA) as a way to generate sufficient signal from low affinity antibodies or low expression antigens was evaluated. As shown in FIG 7A, 7B, 7C and 7D inclusion of the TSA assay allows detection of antigens inaccessible to the direct immunostaining either due to low antibody affinity or low antigen expression.

## Claims

1. A method of performing a circulating tumor cell detection assay comprising:
incubating a patient sample with a plurality of antibodies, wherein the plurality of antibodies comprises an antibody that binds to vimentin, an antibody that binds to another marker expressed on circulating tumor cells, an antibody that binds to a marker expressed on white blood cells, and an antibody that binds to a marker expressed on endothelial cells;
detecting the plurality of antibodies, wherein the detecting comprises generating signals from the plurality of antibodies; and
confirming the presence of a circulating tumor cell in the sample, comprising identifying one or more cells that have a signal from the antibody that binds to vimentin and a signal from the antibody that binds to the another marker expressed on CTC, but not a signal from the antibody that binds to the marker expressed on white blood cells, a signal from the antibody that binds to the marker expressed on endothelial cells, or both, wherein the marker expressed on endothelial cells is CD144, and wherein the another marker expressed on circulating tumor cells comprises CK8, CK18, CK19, or any combination thereof.

2. The method of any one of the previous claims, wherein the marker expressed on white blood cells comprises CD45.

3. A method of simultaneously identifying a circulating tumor cell and a circulating endothelial cell in a patient sample, comprising:
incubating the sample with a plurality of antibodies, wherein the plurality of antibodies comprises an antibody that binds to vimentin, an antibody that binds to CK8/18/19, and an antibody that binds to CD144;
detecting the plurality of antibodies, wherein the detecting comprises generating signals from the plurality of antibodies; and
identifying the presence of a circulating tumor cell and a circulating endothelial cell in the sample comprising:
screening for a circulating tumor cell, wherein the circulating tumor cell has a signal from the antibody that binds to vimentin, a signal from the antibody that binds to CK8/18/19, or both, but not a signal from the antibody that binds to CD144; and
screening for a circulating endothelial cell, wherein the circulating endothelial cell has a signal from the antibody that binds to vimentin, a signal from the antibody that binds to CK8/18/19, or both, and a signal from the antibody that binds to CD144.

4. A method for distinguishing between a circulating tumor cell and a circulating endothelial cell in a patient sample, comprising:
incubating the sample with a plurality of antibodies, wherein the plurality of antibodies comprises an antibody that binds to vimentin, an antibody that binds to CK8/18/19, and an antibody that binds to CD144;
detecting the plurality of antibodies, wherein the detecting comprises generating signals from the plurality of antibodies; and
distinguishing between a circulating tumor cell and a circulating endothelial cell, comprising screening the sample for a circulating tumor cell, wherein the circulating tumor cell has a signal from the antibody that binds to vimentin, a signal from the antibody that binds to CK8/18/19, or both, but not a signal from the antibody that binds to CD144, and screening the sample for a circulating endothelial cell, wherein the circulating endothelial cell has a signal from the antibody that binds to vimentin, a signal from the antibody that binds to CK8/18/19, or both, and a signal from the antibody that binds to CD144.

5. The method of any one of the previous claims, wherein said patient sample is treated to enrich the concentration of circulating tumor cells, preferably wherein the patient sample is treated by filtration.

6. The method of any one of the previous claims, wherein the plurality of antibodies contain one or more detectable labels.

7. The method of claim 6, wherein the one or more detectable labels comprise fluorescent tags.

8. The method of any one of claims 1 to 7, wherein the detecting is indirect immunofluorescence comprising incubating the sample with a plurality of fluorescently labeled secondary antibodies.

9. The method of any one of the previous claims, further comprising correlating the signal specific to the circulating tumor cells with a tumor burden.

10. The method of any one of the previous claims, further comprising isolating the circulating tumor cells from the sample.

## Patentansprüche

1. Verfahren zum Ausführen einer Analyse zum Feststellen von zirkulierenden Tumorzellen, umfassend:
Inkubieren einer Patientenprobe mit einer Vielzahl von Antikörpern, wobei die Vielzahl von Antikörpern einen Antikörper umfasst, der sich an Vimentin bindet, einen Antikörper, der sich an einen anderen Marker bindet, der auf zirkulierenden Tumorzellen exprimiert wird, einen Antikörper, der sich an einen Marker bindet, der auf weißen Blutzellen exprimiert wird, und einen Antikörper, der sich an einen Marker bindet, der auf endothelialen Zellen exprimiert wird;
Feststellen der Vielzahl von Antikörpern, wobei das Feststellen das Erzeugen von Signalen aus der Vielzahl von Antikörpern umfasst; und
Bestätigen des Vorhandenseins einer zirkulierenden Tumorzelle in der Probe, das das Identifizieren von einer oder mehreren Zellen umfasst, die ein Signal vom Antikörper haben, der sich an Vimentin bindet, und ein Signal von dem Antikörper, der sich an einen anderen Marker bindet, der auf CTC exprimiert wird, aber kein Signal von dem Antikörper, der sich an den Marker bindet, der auf weißen Blutzellen exprimiert wird, ein Signal vom Antikörper, der sich an den Marker bindet, der auf endothelialen Zellen exprimiert wird, oder beides, wobei der Marker, der auf endothelialen Zellen exprimiert wird, CD144 ist, und wobei der andere Marker, der auf zirkulierenden Tumorzellen exprimiert wird, CK8, CK18, CK19 oder eine Kombination derselben umfasst.

2. Verfahren nach einem der vorherigen Ansprüche, wobei der Marker, der auf weißen Blutzellen exprimiert wird, CD45 umfasst.

3. Verfahren zum gleichzeitigen Identifizieren einer zirkulierenden Tumorzelle und einer zirkulierenden endothelialen Zelle in einer Patientenprobe, umfassend:
Inkubieren der Probe mit einer Vielzahl von Antikörpern, wobei die Vielzahl von Antikörpern einen Antikörper umfasst, der sich an Vimentin bindet, einen Antikörper, der sich an CK8/18/19 bindet, und einen Antikörper, der sich an CD144 bindet;
Feststellen der Vielzahl von Antikörpern, wobei das Feststellen das Erzeugen von Signalen aus der Vielzahl von Antikörpern umfasst; und
Identifizieren des Vorhandenseins einer zirkulierenden Tumorzelle und einer zirkulierenden endothelialen Zelle in der Probe, umfassend:
Screening auf eine zirkulierende Tumorzelle, wobei die zirkulierende Tumorzelle ein Signal von dem Antikörper hat, der sich an Vimentin bindet, ein Signal von dem Antikörper, der sich an CK8/18/19 bindet, oder beide, aber kein Signal von dem Antikörper, der sich an CD144 bindet; und
Screening auf eine zirkulierende endotheliale Zelle, wobei die zirkulierende endotheliale Zelle ein Signal von dem Antikörper hat, der sich an Vimentin bindet, ein Signal von dem Antikörper, der sich an CK8/18/19 bindet, oder beide, und ein Signal von dem Antikörper, der sich an CD144 bindet.

4. Verfahren zum Unterscheiden zwischen einer zirkulierenden Tumorzellen und einer zirkulierenden endothelialen Zelle in einer Patientenprobe, umfassend:
Inkubieren der Probe mit einer Vielzahl von Antikörpern, wobei die Vielzahl von Antikörpern einen Antikörper umfasst, der sich an Vimentin bindet, einen Antikörper, der sich an CK8/18/19 bindet, und einen Antikörper, der sich an CD144 bindet;
Feststellen der Vielzahl von Antikörpern, wobei das Feststellen das Erzeugen von Signalen aus der Vielzahl von Antikörpern umfasst; und
Unterscheiden zwischen einer zirkulierenden Tumorzelle und einer zirkulierenden endothelialen Zelle, das das Screening der Probe auf eine zirkulierende Tumorzelle umfasst, wobei die zirkulierende Tumorzelle ein Signal von dem Antikörper hat, der sich an Vimentin bindet, ein Signal von dem Antikörper, der sich an CK8/18/19 bindet, oder beide, aber kein Signal von dem Antikörper, der sich an CD144 bindet, und Screening der Probe auf eine zirkulierende endotheliale Zelle, wobei die zirkulierende endotheliale Zelle ein Signal von dem Antikörper hat, der sich an Vimentin bindet, ein Signal von dem Antikörper, der sich an CK8/18/19 bindet, oder beide, und ein Signal von dem Antikörper, der sich an CD144 bindet.

5. Verfahren nach einem der vorherigen Ansprüche, wobei die Patientenprobe behandelt wird, um die Konzentration von zirkulierenden Tumorzellen zu erhöhen, wobei vorzugsweise die Patientenprobe durch Filtrieren behandelt wird.

6. Verfahren nach einem der vorherigen Ansprüche, wobei die Vielzahl von Antikörpern ein oder mehrere nachweisbare Markierungen hat.

7. Verfahren nach Anspruch 6, wobei die eine oder mehreren nachweisbaren Markierungen Fluoreszenz-Tags umfassen.

8. Verfahren nach einem der Ansprüche 1 bis 7, wobei das Feststellen indirekte Immunofluoreszenz ist, die das Inkubieren der Probe mit einer Vielzahl von fluoreszent markierten sekundären Antikörpern umfasst.

9. Verfahren nach einem der vorherigen Ansprüche, das ferner das Korrelieren des für die zirkulierende Tumorzelle spezifischen Signals mit einer Tumorlast umfasst.

10. Verfahren nach einem der vorherigen Ansprüche, das ferner das Isolieren der zirkulierenden Tumorzellen aus der Probe umfasst.

## Revendications

1. Procédé de mis en oeuvre d'un test de détection de cellules tumorales en circulation comprenant les étapes consistant à :
incuber un échantillon de patient avec une pluralité d'anticorps, où la pluralité d'anticorps comprend un anticorps qui se lie à la vimentine, un anticorps qui se lie à un autre marqueur exprimé sur des cellules tumorales en circulation, un anticorps qui se lie à un marqueur exprimé sur les globules blancs, et un anticorps qui se lie à un marqueur exprimé sur des cellules endothéliales ;
détecter la pluralité d'anticorps, où la détection consiste à générer des signaux à partir de la pluralité d'anticorps ; et
confirmer la présence d'une cellule tumorale en circulation dans l'échantillon, comprenant une identification d'une ou plusieurs cellules qui ont un signal provenant de l'anticorps qui se lie à la vimentine et un signal de l'anticorps qui se lie à l'autre marqueur exprimé en CTC, mais pas un signal provenant de l'anticorps qui se lie au marqueur exprimé sur les globules blancs, un signal provenant de l'anticorps qui se lie au marqueur exprimé sur des cellules endothéliales, ou les deux, où le marqueur exprimé sur les cellules endothéliales est CD144, et où l'autre marqueur exprimé sur les cellules tumorales en circulation comprennent CK8, CK18, CK19, ou toute combinaison de ces éléments.

2. Procédé selon l'une quelconque des revendications précédentes, où le marqueur exprimé sur les globules blancs comprend CD45.

3. Procédé d'identification simultanée d'une cellule tumorale en circulation et d'une cellule endothéliale en circulation dans un échantillon de patient, comprenant les étapes consistant à :
incuber l'échantillon avec une pluralité d'anticorps, où la pluralité d'anticorps comprend un anticorps qui se lie à la vimentine, un anticorps qui se lie au CK8/18/19 et un anticorps qui se lie à CD144 ;
détecter la pluralité d'anticorps, où la détection consiste à générer des signaux à partir de la pluralité d'anticorps ; et
identifier la présence d'une cellule tumorale en circulation et d'une cellule endothéliale en circulation dans l'échantillon comprenant les étapes consistant à :
dépister une cellule tumorale en circulation, où la cellule tumorale en circulation a un signal de l'anticorps qui se lie à la vimentine, un signal de l'anticorps qui se lie au CK8/18/19, ou aux deux, mais non un signal provenant de l'anticorps qui se lie au CD144 ; et
dépister une cellule endothéliale en circulation, où la cellule endothéliale en circulation a un signal de l'anticorps qui se lie à la vimentine, un signal de l'anticorps qui se lie au CK8/18/19, ou aux deux, et un signal de l'anticorps qui se lie au CD144.

4. Procédé pour distinguer entre une cellule tumorale en circulation et une cellule endothéliale en circulation dans un échantillon de patient, comprenant les étapes consistant à :
incuber l'échantillon avec une pluralité d'anticorps, où la pluralité d'anticorps comprend un anticorps qui se lie à la vimentine, un anticorps qui se lie au CK8/18/19 et un anticorps qui se lie au CD144 ;
détecter la pluralité d'anticorps, où la détection consiste à générer des signaux à partir de la pluralité d'anticorps ; et
distinguer entre une cellule tumorale en circulation et une cellule endothéliale en circulation, comprenant un dépistage de l'échantillon pour une cellule tumorale en circulation, où la cellule tumorale en circulation a un signal provenant de l'anticorps qui se lie à la vimentine, un signal de l'anticorps qui se lie au CK8/18/19, ou les deux, mais pas un signal de l'anticorps qui se lie au CD144, et dépister l'échantillon pour une cellule endothéliale en circulation, où la cellule endothéliale en circulation a un signal provenant de l'anticorps qui se lie à la vimentine, un signal de l'anticorps qui se lie au CK8/18/19, ou les deux, et un signal de l'anticorps qui se lie au CD144.

5. Procédé selon l'une des revendications précédentes, où ledit échantillon de patient est traité pour enrichir la concentration des cellules tumorales en circulation, de préférence où l'échantillon du patient est traité par filtration.

6. Procédé selon l'une des revendications précédentes, où la pluralité de revendications contient une ou plusieurs étiquettes détectables.

7. Procédé selon la revendication 6, où une ou plusieurs étiquettes détectables comprennent des balises fluorescentes.

8. Procédé selon l'une des revendications 1 à 7, où la détection est l'immunofluorescence indirecte comprenant une incubation de l'échantillon avec une pluralité d'anticorps secondaires étiquetés par fluorescence.

9. Procédé selon l'une quelconque des revendications précédentes, comprenant en outre une corrélation entre le signal spécifique aux cellules tumorales en circulation avec une charge tumorale.

10. Procédé selon l'une des revendications précédentes, comprenant en outre une isolation des cellules tumorales en circulation de l'échantillon.
